# EUROPÄISCHE PATENTSCHRIFT

(11) **EP 0 463 464 B1**
(45) Veröffentlichungstag und Bekanntmachung des Hinweises auf die Patenterteilung: **08.03.1995**
(21) Anmeldenummer: 91109571.9
(22) Anmeldetag: 11.06.1991
(51) Int. Cl.: C07D 213/61

(54) **Verfahren zur Herstellung von 2-Chlor-5-methyl-pyridin**
Process for the preparation of 2-chloro-5-methylpyridine
Procédé pour la préparation de 2-chloro-5-méthylpyridine

(30) Priorität: 23.06.1990 DE 4020052
(43) Veröffentlichungstag der Anmeldung: 02.01.1992
(73) Patentinhaber: BAYER AG, 51368 Leverkusen (DE)
(72) Erfinder: Kaufmann, Dieter, Dr., 38640 Goslar (DE); Jelich, Klaus, Dr., W-5600 Wuppertal (DE)

(56) Entgegenhaltungen:
- EP-A- 0 324 174
- EP-A- 0 370 317
- CHEMICAL AND PHARMACEUTICAL BULLETIN, vol. 36, no. 6, 1988, Seiten 2244 - 2247;H. YAMANAKA et al.: "Site-Selectivity in the Reaction of 3-Substituted Pyridine 1-Oxides with Phosphoryl Chloride"

## Beschreibung

Die Erfindung betrifft ein neues Verfahren zur Herstellung von 2-Chlor-5-methyl-pyridin.

Es ist bekannt, daß man 2-Chlor-5-methyl-pyridin neben 2-Chlor-3-methyl-pyridin, 4-Chlor-3-methyl-pyridin und 3-Chlor-5-methyl-pyridin erhält, wenn man 3-Methyl-pyridin-1-oxid mit Phosphorylchlorid umsetzt (vergl. Weißberger, Chemistry of Heterocyclic Compounds, Pyridine and its Derivatives, Vol. 14, Supplement, Part 2, S. 112). Hauptprodukt dieser Umsetzung ist 4-Chlor-3-methyl-pyridin; der Anteil an 2-Chlor-5-methyl-pyridin liegt im allgemeinen unter 25 %
oder bei 27% vgl. Chem.and Pharm. Bull.Vol.36, No.6, 1988, Seite 2245, Tab. I.

Weiter ist bekannt, daß man 2-Chlor-5-methyl-pyridin erhält, wenn man 3-Methyl-pyridin-1-oxid mit Phosphorylchlorid in Gegenwart einer Basischen organischen Stickstoffverbindung und in Gegenwart eines Verdünnungsmittels umsetzt (vergl. EP-A 324 174).

Die Reaktion von Pyridin-1-oxiden kann auch mit Phosphorsäureesterchloriden oder Phosphorsäureamidchloriden in Gegenwart von Säureakzeptoren durchgeführt werden (vgl. EP-OS 370 371). Die Verwendung von phosphorhaltigen Chlorierungsmitteln bedingt den Anfall phosphorhaltiger Abfälle, deren Entsorgung problematisch ist.

Es wurde nun ein neues Verfahren zur Herstellung von 2-Chlor-5-methyl-pyridin der Formel (I) 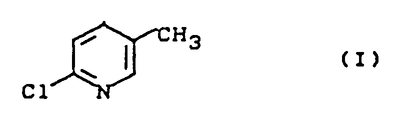
aus 3-Methyl-pyridin-1-oxid der Formel (II) 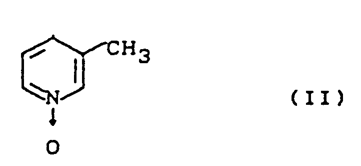
und einem Chlorierungsmittel gefunden, welches dadurch gekennzeichnet ist, daß man die Umsetzung in Gegenwart eines Chlorierungsmittels der allgemeinen Formel (III) 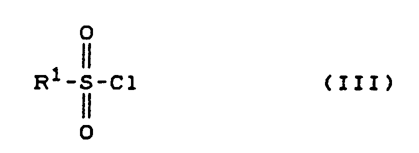
in welcher
- R¹: für Alkyl (C₁-C₆), Halogenalkyl (C₁-C₆), Cycloalkyl (C₃-C₇), gegebenenfalls durch Halogen und/oder Alkyl (C₁-C₆) substituiertes Phenyl oder Naphthyl, NR²R³ oder OR⁴, steht,
worin
- R² und R³: einzeln für Alkyl (C₁-C₆), Cycloalkyl (C₃-C₆) oder Phenyl stehen oder zusammen für Alkandiyl (C₂-C₆) oder Oxaalkandiyl (C₂-C₅) stehen und
- R⁴: für Alkyl (C₁-C₆), Cycloalkyl (C₃-C₇), gegebenenfalls durch Halogen und/oder Alkyl (C₁-C₄) substituiertes Phenyl oder Naphthyl steht.
in Gegenwart einer basischen organischen Stickstoffverbindung und in Gegenwart eines Verdünnungsmittels bei Temperaturen zwischen -20°C und +150°C durchführt und das Reaktionsprodukt in üblicher Weise aufarbeitet.

Es ist als überraschend zu bezeichnen, daß 2-Chlor-5-methyl-pyridin nach dem erfindungsgemäßen Verfahren in guten Ausbeuten erhalten werden kann, da die Herstellung dieser und ähnlicher Verbindungen unter Verwendung von Chlorierungsmitteln der Formel (III) nicht bekannt ist und auch durch bisher bekannt gewordene Verfahren nicht nahegelegt wird.

Der Vorteil des erfindungsgemäßen Verfahrens gegenüber dem bekannten Stand der Technik liegt insbesondere darin, daß die Chlorierungsmittel der Formel (III) und ihre Umwandlungsprodukte ohne größere Probleme entsorgt werden können.

Das erfindungsgemäße Verfahren stellt somit eine wertvolle Bereicherung des Standes der Technik dar.

Der Reaktionsablauf beim erfindungsgemäßen Verfahren kann beispielsweise durch das nachstehende Formelschema skizziert werden:

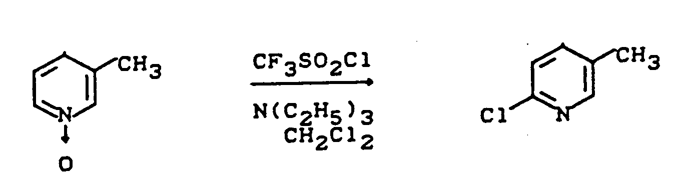

Die Ausgangsverbindung der Formel (II) ist bereits bekannt (vergl. J. Am. Chem. Soc. 76 (1954), 1286-1291).

Die beim erfindungsgemäßen Verfahren zu verwendenden Chlorierungsmittel sind durch die Formel (III) allgemein definiert. In der Formel (III) stehen vorzugsweise
- R¹: für Alkyl (C₁-C₄), Perfluoralkyl (C₁-C₆), Perchloralkyl (C₁-C₆), Cycloalkyl (C₅-C₆), Phenyl, durch Halogen und/oder Alkyl (C₁-C₄) substituiertes Phenyl, Naphthyl, durch Halogen und/oder Alkyl (C₁-C₄) substituiertes Naphthyl oder für NR²R³ oder OR⁴
worin
- R² und R³: einzeln für Methyl, Ethyl, Propyl, Isopropyl, Butyl, Isobutyl, Cyclopentyl, Cyclohexyl oder Phenyl stehen oder zusammen für Butan-1,4-diyl (Tetramethylen), Pentan-1,5-diyl (Pentamethylen) oder 3-Oxa-pentan-1,5-diyl (-CH₂CH₂-O-CH₂CH₂-) stehen,
und worin
- R⁴: für Alkyl (C₁-C₆), Cycloalkyl (C₅-C₆), gegebenenfalls durch Chlor und/oder Methyl substituiertes Phenyl oder Naphthyl steht.
Ganz besonders bevorzugte Chlorierungsmittel der Formel (III) sind diejenigen, bei denen
- R¹: für Perfluoralkyl (C₁-C₆), Perchloralkyl (C₁-C₆), Phenyl, Naphthyl oder Dialkylamino NR²R³ steht,
worin
- R² und R³: für Methyl, Ethyl, Propyl oder Butyl stehen.

Als Beispiele für die Chlorierungsmittel der Formel (III) seien genannt:
Trifluormethyl-sulfonylchlorid
Nonafluorbutyl-sulfonylchlorid
Perfluorhexyl-sulfonylchlorid
Trichlormethyl-sulfonylchlorid
Phenyl-sulfonylchlorid
4-Chlorphenyl-sulfonylchlorid
2-Chlorphenyl-sulfonylchlorid
2,5-Dichlorphenyl-sulfonylchlorid
2-Methylphenyl-sulfonylchlorid
1-Naphthyl-sulfonylchlorid
2-Naphthyl-sulfonylchlorid
1,5-Naphthalin-di-sulfonylchlorid
2,6-Naphthalin-di-sulfonylchlorid
N,N-Dimethyl-sulfamoylchlorid
N,N-Diethyl-sulfamoylchlorid
N,N-Dipropyl-sulfamoylchlorid
N,N-Dibutyl-sulfamoylchlorid
Die Verbindungen der Formel (III) sind bekannt und/oder können nach an sich bekannten Verfahren hergestellt werden (vergl. dazu Houben-Weyl, Band 9, G. Thieme Verlag 1955, S. 343 ff, S. 557 ff).

Falls man beim erfindungsgemäßen Verfahren mit einem Alkyl- oder Arylsulfonylchlorid als Chlorierungsmittel (III) arbeitet,
so steht
- R¹: vorzugsweise für Methyl, Ethyl, n-Propyl, i-Propyl, n-Butyl, i-Butyl, tert.-Butyl, n-Amyl, i-Amyl, Cyclopentyl, Cyclohexyl, Trifluormethyl, Pentafluorethyl, Heptafluorpropyl, Nonylfluorbutyl, Trichlormethyl, Phenyl, Toluyl, Xyloyl, Chlorphenyl, Dichlorphenyl, Naphthyl, 1,5-Naphthdiyl oder 2,6-Naphthdiyl.

Falls man beim erfindungsgemäßen Verfahren mit einem Chlorsulfonsäurealkyl- oder -arylester als Chlorierungsmittel (III) arbeitet, so steht
- R¹: für die Gruppierung OR⁴, wobei
- R⁴: vorzugsweise für Methyl, Ethyl, n-Propyl, i-Propyl, n-Butyl, i-Butyl, tert.-Butyl, n-Amyl, i-Amyl, Cyclopentyl, Cyclohexyl, Phenyl, Tolyl, Xylyl, Chlorphenyl, Naphthyl steht.

Falls man beim erfindungsgemäßen Verfahren mit einem Sulfamoylchlorid als Chlorierungsmittel (III) arbeitet, so steht
- R¹: für die Gruppierung NR²R³, wobei
- R² und R³: einzeln vorzugsweise für C₁-C₆-Alkyl, C₅-C₆-Cycloalkyl oder Phenyl stehen oder wobei R² und R³ zusammen für C₂-C₆-Alkandiyl oder C₂-C₅-Oxaalkandiyl stehen.

Das erfindungsgemäße Verfahren wird in Gegenwart einer basischen Stickstoffverbindung durchgeführt. Bevorzugte basische organische Stickstoffverbindungen sind Dialkylamine, wie z.B. Diethylamin, Dipropylamin, Diisopropylamin, Dibutylamin, Diisobutylamin und Di-sec-butylamin, Trialkylamine, wie z.B. Trimethylamin, Triethylamin, Tripropylamin und Tributylamin, Methyldiisopropylamin, Ethyldiisopropylamin, Dialkyl-cycloalkylamine, wie z.B. Dimethyl-cyclopentylamin, Diethyl-cyclopentylamin, Dimethyl-cyclohexylamin und Diethyl-cyclohexylamin, Dialkyl-aralkylamine, wie z.B. Dimethyl-benzylamin und Diethyl-benzylamin sowie Dialkylarylamine, wie z.B. Dimethylanilin.

Besonders bevorzugte basische organische Stickstoffverbindungen sind Trialkylamine, wie z.B. Trimethylamin, Triethylamin, Tripropylamin, Tributylamin, Methyldiisopropylamin und Ethyldiisopropylamin.

Das erfindungsgemäße Verfahren wird in Gegenwart eines Verdünnungsmittels durchgeführt. Als Verdünnungsmittel kommen praktisch alle inerten organischen Lösungsmittel in Frage. Hierzu gehören vorzugsweise gegebenenfalls halogenierte Kohlenwasserstoffe, wie Pentan, Hexan, Heptan, Octan, Cyclohexan, Methylcyclohexan, Petrolether, Benzin, Ligroin, Benzol, Toluol, Xylol, Methylenchlorid, Chloroform, Tetrachlormethan, 1,2-Dichlorethan, 1,1,2-Trichlorethan, 1,2-Dichlorpropan, 1,2,3-Trichlorpropan, Chlorbenzol und Dichlorbenzol, Ether, wie Diethylether, Dipropylether, Diisopropylether, Dibutylether, Methyl-tert.-butylether, Methyl-tert.-amylether, Glycoldimethylether, Diglycoldimethylether, Tetrahydrofuran, Dioxan und Anisol, Ketone, wie Aceton, Methylethylketon, Diethylketon, Methyl-isopropylketon und Methyl-isobutylketon, Ester, wie Essigsäuremethylester, Essigsäureethylester, Essigsäurepropylester, Essigsäurebutylester und Essigsäureamylester, Nitrile, wie Acetonitril und Propionitril, Amide wie Dimethylformamid, Dimethylacetamid und N-Methyl-pyrrolidon, sowie Dimethylsulfoxid und Sulfolan.

Die Reaktionstemperaturen können bei dem erfindungsgemäßen Verfahren in einem größeren Bereich variiert werden. Im allgemeinen arbeitet man bei Temperaturen zwischen -20°C und +150°C, vorzugsweise bei Temperaturen zwischen 0°C und +80°C.

Das erfindungsgemäße Verfahren wird im allgemeinen unter Normaldruck durchgeführt. Es ist jedoch auch möglich, unter erhöhtem oder vermindertem Druck zwischen 0,1 und 10 bar zu arbeiten.

Zur Durchführung des erfindungsgemäßen Verfahrens setzt man auf 1 Mol 3-Methyl-pyridin-1-oxid der Formel (II) im allgemeinen zwischen 1 und 10 Mol, vorzugsweise zwischen 1,5 und 4,0 Mol, Chlorierungsmittel der Formel (III) und ebenfalls zwischen 1 und 10 Mol, vorzugsweise zwischen 1,5 und 4,0 Mol, der basischen organischen Stickstoffverbindung ein.

In einer bevorzugten Ausführungsform des erfindungsgemäßen Verfahrens werden das 3-Methyl-pyridin-1-oxid und die basische organische Stickstoffverbindung in einem Verdünnungsmittel vorgelegt, und das Chlorierungsmittel der Formel (III) wird dann unter Rühren langsam eindosiert. Das komplette Reaktionsgemisch wird dann gegebenenfalls noch weiter bis zum Ende der Umsetzung gerührt.

Die Aufarbeitung kann auf übliche Weise durchgeführt werden. Beispielsweise wird das Reaktionsgemisch mit Wasser verdünnt, gegebenenfalls mit Natronlauge neutral gestellt und die organische Phase abgetrennt. Aus der wäßrigen Phase wird mit einem organischen Lösungsmittel, wie z.B. Methylenchlorid, weiteres Produkt extrahiert. Die vereinigten organischen Phasen werden getrocknet und filtriert; vom Filtrat wird das Lösungsmittel im Wasserstrahlvakuum sorgfältig abdestilliert.

Der verbleibende Rückstand enthält im wesentlichen das Produkt der Formel (I), welches auf übliche Weise, beispielsweise durch Vakuumdestillation, weiter gereinigt werden kann.

Das nach dem erfindungsgemäßen Verfahren herstellbare 2-Chlor-5-methyl-pyridin ist als Zwischenprodukt für Pharmazeutika bekannt (vergl. DE-A 2 812 585), kann aber auch als Zwischenprodukt für Insektizide eingesetzt werden (vergl. DE-A 2 630 046).

### Herstellungsbeispiel 1

### 2-Chlor-5-methyl-pyridin

Zu einer Lösung von 5,5 g (50 mmol) 3-Methylpyridin-1-oxid und 7,6 g (75 mmol) Triethylamin in 80 ml Methylenchlorid wird unter Stickstoff eine Lösung von 12,6 g (75 mmol) Trifluormethansulfonsäurechlorid in 20 ml Methylenchlorid in 20 min getropft. Anschließend wird noch 12 Stunden unter Rückfluß erhitzt, dann abgesaugt und der Filterkuchen mit Methylenchlorid gewaschen. Die Methylenchlorid-Lösung wird mit konz. Salzsäure verrührt, die wäßrige Phase abgetrennt, mit Natronlauge neutral gestellt und mit Methylenchlorid extrahiert. Nach Abdestillieren des Lösungsmittels verbleibt ein Rückstand, der gaschromatographisch analysiert wird.
- Ausbeute:: 3,82 g (60 %) eines Gemisches aus 80 % 2-Chlor-5-methylpyridin und 20 % 2-Chlor-3-methylpyridin.

Das reine 2-Chlor-5-methyl-pyridin kann durch fraktionierte Destillation abgetrennt werden.

### Herstellungsbeispiel 2

### 2-Chlor-5-methyl-pyridin

Zu einer Lösung von 21,8 g (0,2 mol) 3-Methylpyridin-1-oxid und 40,4 g (0,4 mol) Triethylamin in 150 ml Methylenchlorid wird unter Stickstoff eine Lösung von 76,2 g (0,4 mol) p-Toluolsulfonsäurechlorid in 150 ml Methylenchlorid in 70 min getropft. Anschließend wird noch 12 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation bei einem pH-Wert von 6 unterworfen. Das Destillat wird dreimal mit je 100 ml Methylenchlorid extrahiert. Nach Abdestillieren des Lösungsmittels verbleibt ein Rückstand, der gaschromatographisch analysiert wird.
- Ausbeute:: 13,2 g (52 %) eines Gemisches aus 70 % 2-Chlor-5-methylpyridin und 30 % 2-Chlor-3-methylpyridin.

Das reine 2-Chlor-5-methyl-pyridin kann durch fraktionierte Destillation abgetrennt werden.

### Herstellungsbeispiel 3

### 2-Chlor-5-methyl-pyridin

Zu einer Lösung von 5,5 g (50 mmol) 3-Methylpyridin-1-oxid und 10,1 g (0,1 mol) Triethylamin in 40 ml Chlorbenzol wird unter Stickstoff eine Lösung von 20 g (0,1 mol) N,N-Dipropylsulfamoylchlorid in 60 ml Chlorbenzol getropft. Anschließend wird noch 3 Stunden auf 70°C erhitzt, dann abgesaugt, der Filterkuchen mit Chlorbenzol gewaschen und die flüssige Phase mit konz. Salzsäure extrahiert. Die wäßrige Phase wird mit Natronlauge neutral gestellt und mit Toluol extrahiert. Nach Abdestillieren des Lösungsmittels verbleibt ein Rückstand, der gaschromatographisch analysiert wird.
- Ausbeute:: 3,37 g (53 %) eines Gemisches aus 80 % 2-Chlor-5-methylpyridin und 20 % 2-Chlor-3-methylpyridin.

Das reine 2-Chlor-5-methyl-pyridin kann durch fraktionierte Destillation abgetrennt werden.

## Patentansprüche

1. Verfahren zur Herstellung von 2-Chlor-5-methyl-pyridin der Formel (I) 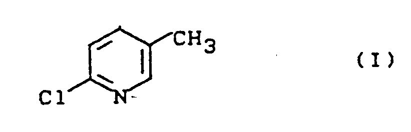 aus 3-Methyl-pyridin-1-oxid der Formel (II) 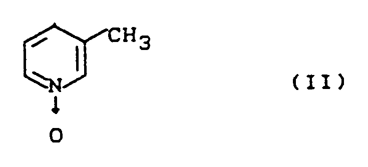 und einem Chlorierungsmittel, dadurch gekennzeichnet, daß man die Umsetzung in Gegenwart eines Chlorierungsmittels der allgemeinen Formel (III) 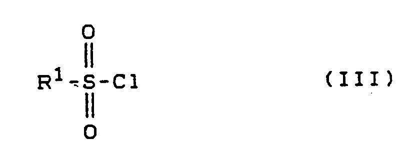 in welcher
R¹ für Alkyl (C₁-C₆), Halogenalkyl (C₁-C₆), Cycloalkyl (C₃-C₇), gegebenenfalls durch Halogen und/oder Alkyl (C₁-C₆) substituiertes Phenyl oder Naphthyl, NR²R³ oder OR⁴, steht,
worin
R² und R³ einzeln für Alkyl (C₁-C₆), Cycloalkyl (C₃-C₆) oder Phenyl stehen oder zusammen für Alkandiyl (C₂-C₆) oder Oxaalkandiyl (C₂-C₅) stehen und
R⁴ für Alkyl (C₁-C₆), Cycloalkyl (C₃-C₇), gegebenenfalls durch Halogen und/oder Alkyl (C₁-C₄) substituiertes Phenyl oder Naphthyl steht.
in Gegenwart einer basischen organischen Stickstoffverbindung und in Gegenwart eines Verdünnungsmittels bei Temperaturen zwischen -20°C und +150°C durchführt und das Reaktionsprodukt in üblicher Weise aufarbeitet.

2. Verfahren gemäß Anspruch 1, dadurch gekennzeichnet, daß man als Chlorierungsmittel ein Alkyl- oder Arylsulfonylchlorid der Formel (III) einsetzt, in welcher
R¹ für Methyl, Ethyl, n-Propyl, i-Propyl, n-Butyl, i-Butyl, tert.-Butyl, n-Amyl, i-Amyl, Cyclopentyl, Cyclohexyl, Trifluormethyl, Pentafluorethyl, Heptafluorpropyl, Nonafluorbutyl, Trichlormethyl, Phenyl, Toluyl, Xyloyl, Chlorphenyl, Dichlorphenyl, Naphthyl, 1,5-Naphthdiyl oder 2,6-Naphthdiyl steht.

3. Verfahren gemäß Anspruch 1, dadurch gekennzeichnet, daß man als Chlorierungsmittel (III) einen Chlorsulfonsäurealkyl- oder -arylester einsetzt, wobei in der Formel (III) der Rest R¹ für die Gruppierung OR⁴ steht, in welcher
R⁴ für Methyl, Ethyl, n-Propyl, i-Propyl, n-Butyl, i-Butyl, tert.-Butyl, n-Amyl, i-Amyl, Cyclopentyl, Cyclohexyl, Phenyl, Toluyl, Xyloyl, Chlorphenyl, Naphthyl steht.

4. Verfahren gemäß Anspruch 1, dadurch gekennzeichnet, daß man als Chlorierungsmittel ein Sulfamoylchlorid der Formel (III) einsetzt, in welcher
R¹ für die Gruppierung NR²R³ steht, worin
R² und R³ einzeln für C₁-C₆-Alkyl, C₅-C₆-Cycloalkyl oder Phenyl stehen oder zusammen für C₂-C₆-Alkandiyl oder C₂-C₅-Oxaalkandiyl stehen.

5. Verfahren gemäß Anspruch 1 bis 4, dadurch gekennzeichnet, daß man in Gegenwart einer basischen organischen Stickstoffverbindung der folgenden Reihe arbeitet: Dialkylamine, Trialkylamine, Dialkylcycloalkylamine, Dialkyl-aralkylamine sowie Dialkylarylamine.

6. Verfahren gemäß Anspruch 1 bis 5, dadurch gekennzeichnet, daß man in Gegenwart einer basischen organischen Stickstoffverbindung der folgenden Reihe arbeitet: Diethylamin, Dipropylamin, Diisopropylamin, Dibutylamin, Diisobutylamin, Di-sec-butylamin, Trimethylamin, Triethylamin, Tripropylamin, Tributylamin, Methyldiisopropylamin, Ethyldiisopropylamin, Dimethyl-cyclopentylamin, Diethyl-cyclopentylamin, Dimethyl-cyclohexylamin, Dimethylbenzylamin, Diethylbenzylamin, Dimethylanilin.

7. Verfahren gemäß Anspruch 1 bis 6, dadurch gekennzeichnet, daß man in Gegenwart eines inerten organischen Lösungsmittels als Verdünnungsmittel arbeitet.

8. Verfahren gemäß Anspruch 1 bis 7, dadurch gekennzeichnet, daß man in Gegenwart von Methylenchlorid, Ethylenchlorid, 1,1,2-Trichlorethan, 1,2-Dichlorpropan, 1,2,3-Trichlorpropan oder Chlorbenzol als Verdünnungsmittel arbeitet.

9. Verfahren gemäß Anspruch 1 bis 8, dadurch gekennzeichnet, daß man bei einer Temperatur zwischen 0°C und +80°C arbeitet.

10. Verfahren gemäß Anspruch 1 bis 9, dadurch gekennzeichnet, daß man auf 1 Mol 3-Methyl-pyridin-1-oxid der Formel (II) zwischen 1 und 10 Mol Chlorierungsmittel der Formel (III) und zwischen 1 und 10 Mol der basischen organischen Stickstoffverbindung einsetzt.

11. Verfahren gemäß Anspruch 1 bis 10, dadurch gekennzeichnet, daß man das 3-Methyl-pyridin-1-oxid der Formel (II) und die basische Stickstoffverbindung in einem Verdünnungsmittel vorlegt und das Chlorierungsmittel der Formel (III) unter Rühren langsam eindosiert und das komplette Reaktionsgemisch gegebenenfalls noch weiter bis zum Ende der Umsetzung rührt.

## Claims

1. Process for the preparation of 2-chloro-5-methyl-pyridine of the formula (I) 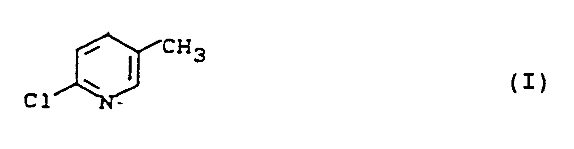 from 3-methyl-pyridine-1-oxide of the formula (II) 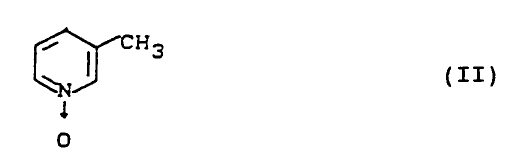 and a chlorinating agent, characterized in that the reaction is carried out in the presence of a chlorinating agent of the general formula (III) 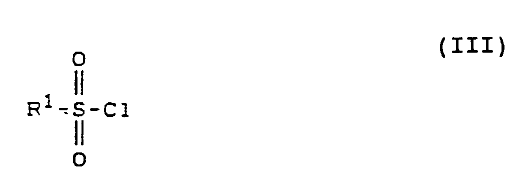 in which
R¹ represents alkyl (C₁-C₆), halogenoalkyl (C₁-C₆), cycloalkyl (C₃-C₇), phenyl or naphthyl which is optionally substituted by halogen and/or alkyl (C₁-C₆), NR²R³ or OR⁴,
in which
R² and R³ individually represent alkyl (C₁-C₆), cycloalkyl (C₃-C₆) or phenyl or together represent alkanediyl (C₂-C₆) or oxaalkanediyl (C₂-C₅) and
R⁴ represents alkyl (C₁-C₆), cycloalkyl (C₃-C₇), phenyl or naphthyl which is optionally substituted by halogen and/or alkyl (C₁-C₄),
in the presence of a basic organic nitrogen compound and in the presence of a diluent at temperatures between -20°C and +150°C, and the reaction product is worked up in the customary manner.

2. Process according to Claim 1, characterized in that an alkyl- or arylsulphonyl chloride of the formula (III) is used as the chlorinating agent, in which
R¹ represents methyl, ethyl, n-propyl, i-propyl, n-butyl, i-butyl, tert.-butyl, n-amyl, i-amyl, cyclopentyl, cyclohexyl, trifluoromethyl, pentafluoroethyl, heptafluoropropyl, nonafluorobutyl, trichloromethyl, phenyl, toluyl, xyloyl, chlorophenyl, dichlorophenyl, naphthyl, 1,5-naphthdiyl or 2,6-naphthdiyl.

3. Process according to Claim 1, characterized in that an alkyl or aryl chlorosulphonate is used as the chlorinating agent (III), where in the formula (III) the radical R¹ represents the group OR⁴, in which
R⁴ represents methyl, ethyl, n-propyl, i-propyl, n-butyl, i-butyl, tert.-butyl, n-amyl, i-amyl, cyclopentyl, cyclohexyl, phenyl, toluyl, xyloyl, chlorophenyl or naphthyl.

4. Process according to Claim 1, characterized in that a sulphamoyl chloride of the formula (III) is used as the chlorinating agent, in which
R¹ represents the group NR²R³, in which
R² and R³ individually represent C₁-C₆-alkyl, C₅-C₆-cycloalkyl or phenyl or together represent C₂-C₆-alkanediyl or C₂-C₅-oxaalkanediyl.

5. Process according to Claims 1 to 4, characterized in that the reaction is carried out in the presence of a basic organic nitrogen compound from the following series: dialkylamines, trialkylamines, dialkylcycloalkylamines, dialkyl-aralkylamines and dialkylarylamines.

6. Process according to Claims 1 to 5, characterized in that the reaction is carried out in the presence of a basic organic nitrogen compound from the following series: diethylamine, dipropylamine, diisopropylamine, dibutylamine, diisobutylamine, di-sec-butylamine, trimethylamine, triethylamine, tripropylamine, tributylamine, methyldiisopropylamine, ethyldiisopropylamine, dimethyl-cyclopentylamine, diethyl-cyclopentylamine, dimethyl-cyclohexylamine, dimethylbenzylamine, diethyl-benzylamine and dimethylaniline.

7. Process according to Claims 1 to 6, characterized in that the reaction is carried out in the presence of an inert organic solvent as the diluent.

8. Process according to Claims 1 to 7, characterised in that the reaction is carried out in the presence of methylene chloride, ethylene chloride, 1,1,2-trichloroethane, 1,2-dichloropropane, 1,2,3-trichloropropane or chlorobenzene as diluent.

9. Process according to Claims 1 to 8, characterized in that the reaction is carried out at a temperature between 0°C and + 80°C.

10. Process according to Claims 1 to 9, characterized in that between 1 and 10 moles of chlorinating agent of the formula (III) and between 1 and 10 moles of the basic organic nitrogen compound are employed per mole of 3-methyl-pyridine-1-oxide of the formula (II).

11. Process according to Claims 1 to 10, characterized in that the 3-methyl-pyridine-1-oxide of the formula (II) and the basic nitrogen compound are initially introduced in a diluent and the chlorinating agent of the formula (III) is slowly metered in with stirring and the complete reaction mixture is optionally further stirred until the reaction is complete.

## Revendications

1. Procédé de production de 2-chloro-5-méthylpyridine de formule (I) 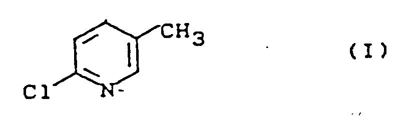 à partir de 1-oxyde de 3-méthylpyridine de formule (II) 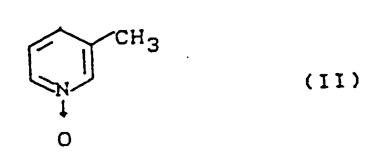 et d'un agent de chloration, caractérisé en ce qu'on conduit la réaction en présence d'un agent de chloration de formule générale (III) 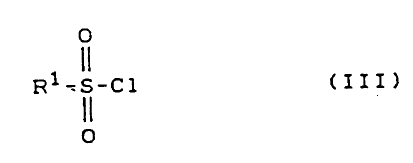 dans laquelle
R¹ est un groupe alkyle (en C₁ à C₆), halogénalkyle (en C₁ à C₆), cycloalkyle (en C₃ à C₇), un groupe phényle ou naphtyle éventuellement substitué par un halogène et/ou par un radical alkyle (en C₁ à C₆), un groupe NR²R³ ou OR⁴,
où
R² et R³ représentent, individuellement, un groupe alkyle (en C₁ à C₆), cycloalkyle (en C₃ à C₆) ou phényle ou forment conjointement un groupe alcanediyle (en C₂ à C₆) ou oxaalcanediyle (en C₂ à C₅) et
R⁴ est un groupe alkyle (en C₁ à C₆), cycloalkyle (en C₃ à C₇), un groupe phényle ou naphtyle éventuellement substitué par un halogène et/ou par un radical alkyle (en C₁ à C₄),
en présence d'un composé azoté organique basique et en présence d'un diluant, à des températures comprises entre -20°C et +150°C, et on traite le produit de réaction d'une manière classique.

2. Procédé suivant la revendication 1, caractérisé en ce qu'on utilise comme agent de chloration un chlorure d'alkyl- ou d'arylsulfonyle de formule (III), dans laquelle
R¹ est un groupe méthyle, éthyle, n-propyle, iso-propyle, n-butyle, iso-butyle, tertio-butyle, n-amyle, iso-amyle, cyclopentyle, cyclohexyle, trifluorométhyle, pentafluoréthyle, heptafluoropropyle, nonafluorobutyle, trichlorométhyle, phényle, toluyle, xyloyle, chlorophényle, dichlorophényle, naphtyle, 1,5-naphtdiyle ou 2,6-naphtdiyle.

3. Procédé suivant la revendication 1, caractérisé en ce qu'on utilise comme agent de chloration (III) un ester alkylique ou arylique d'acide chlorosulfonique, auquel cas dans la formule (III), le reste R¹ représente le groupement OR⁴, dans lequel
R⁴ est un groupe méthyle, éthyle, n-propyle, iso-propyle, n-butyle, iso-butyle, tertio-butyle, n-amyle, iso-amyle, cyclopentyle, cyclohexyle, phényle, toluyle, xyloyle, chlorophényle, naphtyle.

4. Procédé suivant la revendication 1, caractérisé en ce qu'on utilise comme agent de chloration un chlorure de sulfamoyle de formule (III), dans laquelle
R¹ représente le groupement NR²R³, où
R² et R³ représentent individuellement un groupe alkyle en C₁ à C₆, cycloalkyle en C₅ ou C₆ ou phényle ou forment conjointement un groupe alcanediyle en C₂ à C₆ ou oxa-alcanediyle en C₂ à C₅.

5. Procédé suivant les revendications 1 à 4, caractérisé en ce qu'on opère en présence d'un composé azoté organique basique de la série suivante : dialkylamines, trialkylamines, dialkylcycloalkylamines, dialkylaralkylamines ainsi que dialkylarylamines.

6. Procédé suivant les revendications 1 à 5, caractérisé en ce qu'on opère en présence d'un composé azoté organique basique de la série suivante : diéthylamine, dipropylamine, diisopropylamine, dibutylamine, diisobutylamine, di-sec.-butylamine, triméthylamine, triéthylamine, tripropylamine, tributylamine, méthyldiisopropylamine, éthyldiisopropylamine, diméthylcyclopentylamine, diéthylcyclopentylamine, diméthylcyclohexylamine, diméthylbenzylamine, diéthylbenzylamine, diméthylaniline.

7. Procédé suivant les revendications 1 à 6, caractérisé en ce qu'on opère en présence d'un solvant organique inerte utilisé comme diluant.

8. Procédé suivant les revendications 1 à 7, caractérisé en ce qu'on opère en présence de chlorure de méthylène, de chlorure d'éthylène, de 1,1,2-trichloréthane, de 1,2-dichloropropane, de 1,2,3-trichloropropane ou de chlorobenzène utilisé comme diluant.

9. Procédé suivant les revendications 1 à 8, caractérisé en ce qu'on opère à une température comprise entre 0°C et +80°C.

10. Procédé suivant les revendications 1 à 9, caractérisé en ce qu'on utilise, par mole de 1-oxyde de 3-méthylpyridine de formule (II), entre 1 et 10 moles d'agent de chloration de formule (III) et entre 1 et 10 moles du composé azoté organique basique.

11. Procédé suivant les revendications 1 à 10, caractérisé en ce qu'on charge préalablement le 1-oxyde de 3-méthylpyridine de formule (II) et le composé azoté basique dans un diluant, et on introduit lentement en agitant l'agent de chloration de formule (III) et, le cas échéant, on continue d'agiter le mélange réactionnel entier jusqu'à la fin de la réaction.
